Europäisches Patentamt

**European Patent Office**

Office européen des brevets

(19)

(11) Veröffentlichungsnummer: **0 143 750**
**B1**

(12) # EUROPÄISCHE PATENTSCHRIFT

(45) Veröffentlichungstag der Patentschrift:
**22.04.87**

(51) Int. Cl.⁴: **C 07 C 143/55**

(21) Anmeldenummer: **84810570.6**

(22) Anmeldetag: **22.11.84**

(54) Verfahren zur Herstellung von 4-Nitrotoluol-2-sulfonsäure.

(30) Priorität: **28.11.83 CH 6350/83**

(43) Veröffentlichungstag der Anmeldung:
**05.06.85 Patentblatt 85/23**

(45) Bekanntmachung des Hinweises auf die Patenterteilung:
**22.04.87 Patentblatt 87/17**

(84) Benannte Vertragsstaaten:
**BE CH DE FR GB IT LI NL**

(56) Entgegenhaltungen:
**EP - A - 0 083 555**
**DD - A - 140 206**

**CHEMICAL ABSTRACTS, Band 93, Nr. 7, 18. August 1980, Seite 931, Zusammenfassung Nr. 71290a, COLUMBUS, OHIO, (US).**

(73) Patentinhaber: **CIBA-GEIGY AG, Klybeckstrasse 141, CH-4002 Basel (CH)**

(72) Erfinder: **Seifert, Gottfried, Mühlemattweg 20, CH-4312 Magden (CH)**
Erfinder: **Stäubli, Sebastian, Lanzenbergstrasse 17, CH-4312 Magden (CH)**
Erfinder: **Wieland, Josef Hermann, Dr., Holbeinstrasse 65, D-7880 Bad Säckingen (DE)**
Erfinder: **Regenass, Willy, Dr., Weinhagstrasse 6, CH-4132 Muttenz (CH)**

## Beschreibung

Die vorliegende Erfindung betrifft ein Verfahren zur Herstellung von 4-Nitrotoluol-2-sulfonsäure durch Sulfonierung von 4-Nitrotoluol mit Oleum.

4-Nitrotoluol-2-sulfonsäure ist ein wichtiges Zwischenprodukt für die Herstellung von optischen Aufhellern, von dem jährlich tausende von Tonnen hergestellt werden. Es dient hauptsächlich als Ausgangsprodukt für die Herstellung von 4,4'-Dinitrostilben-2,2'-disulfonsäure, die ihrerseits zur Herstellung einer Vielzahl von optischen Aufhellern auf Cyanurchloridbasis vom Typ der 4,4'-Bis-([1,3,5-triazin-2-yl]-amino)-stilben-2,2'-disulfonsäuren eingesetzt wird. Weitere beträchtliche Mengen an 4-Nitrotoluol-2-sulfonsäure werden für die Synthese von optischen Aufhellern mit anderen Grundstrukturen sowie von verschiedenen Farbstoffen benötigt.

Die grosstechnische Herstellung von 4-Nitrotoluol-2-sulfonsäure erfolgt bisher praktisch ausschliesslich durch Sulfonierung von 4-Nitrotoluol mit 20-25%igem Oleum. bei diesem Verfahren fallen jedoch grosse Mengen an Abfallschwefelsäure an, welche vom ökologischen Standpunkt aus unerwünscht ist. Ausserdem stellt die Wiederaufarbeitung oder Beseitigung der sauren Mutterlaugen ein technisches Problem dar und belastet das Verfahren auch von der ökonomischen Seite her. Es fehlte daher nicht an Versuchen, die Nachteile dieses Sulfonierungsverfahrens zu überwinden. Insbesondere wurde versucht, 4-Nitrotoluol direkt mit $SO_3$ zu sulfonieren.

In der US-A-3 840 591 ist ein Verfahren zur Herstellung von 4-Nitrotoluol-2-sulfonsäure beschrieben, bei dem geschmolzenes 4-Nitrotoluol direkt mit einer Mischung aus $SO_3$ und einem Inertgas sulfoniert wird. Bei diesem Verfahren entstehen jedoch komplexe farbige Produkte, die sich im Endprodukt anreichern. Das dunkel gefärbte Produkt muss daher relativ aufwendigen Reinigungsoperationen unterworfen werden.

Weiterhin ist aus der DE-A-2 353 918 und der DE-A-2 354 097 ein Verfahren zur Darstellung von aromatischen Sulfonsäuren durch Behandlung von aromatischen Kohlenwasserstoffen mit gasförmigem $SO_3$ bekannt, bei dem die Reaktion unter ständig aufrechterhaltenem grossem Überschuss von aromatischem Kohlenwasserstoff als Wärmeträgermedium unter Rückflussbedingungen stattfindet, wobei gemäss der DE-A-2 354 097 als Wärmeträgermedium eine inerte, die aromatische Substanz und die aus ihr gebildete Sulfonsäure lösende Verbindung eingesetzt wird. Die Reaktionstemperatur, die über den Druck geregelt wird, liegt zwischen 20 und 100°C und stimmt mit der Rückflusstemperatur überein.

Nach dem oben beschriebenen Verfahren ist aus physikalischen Gegebenheiten im Falle der Sulfonierung von 4-Nitrotoluol (Siedepunkt 105°C/12 mbar) das lösungsmittelfreie Verfahren technisch nur mit erheblichem Aufwand durchführbar. Auch die vorgeschlagene Verwendung von Lösungsmitteln bedeutet einen zusätzlichen technischen Aufwand, sofern überhaupt im fraglichen Temperaturbereich gegen $SO_3$ genügend stabile organische Lösungsmittel zur Verfügung stehen.

Aus der FR-A-1 555 394, Beispiel 2, ist die Herstellung von 4-Nitrotoluol-2-sulfonsäure bekannt, wobei dem geschmolzenen 4-Nitrotoluol bei 80 bis 90°C gasförmiges $SO_3$ zugeführt wird. Anschliessend wird die Temepratur auf 115 - 120°C gesteigert und 8 Stunden beibehalten. Die heisse Schmelze ergibt nach Ablassen in Wasser eine dunkelbraune Lösung der 4-Nitrotoluol-2-sulfonsäure. Entsprechend CH-A-478 772, Spalte 2, Absatz 3 kann die Sulfonierung auch ohne Lösungsmittel durch Einleiten von gasförmigem, stark mit indifferenten Gasen, wie Stickstoff, verdünntem Schwefeltrioxid oder oxidierten Restgasen z.B. aus einer Kontakt-Schwefelsäureanlage in den flüssigen oder geschmolzenen Nitroaromaten durchgeführt werden.

Die Sulfonierung einer Schmelze von 4-Nitrotoluol mit reinem gasförmigem $SO_3$ (eine solche Sulfonierung ist z.B. in der DE-A-2 837 549 beschrieben) kommt praktisch nicht in Frage, da wegen des hohen Schmelzpunktes der 4-Nitrotoluol-2-sulfonsäure die Sulfonierungstemperatur in deren Zersetzungsbereich angehoben werden müsste.

Wenn bei der Sulfonierung mit gasförmigem $SO_3$ nicht bis zum vollständigen 4-Nitrotoluol-Umsatz gefahren wird, kann die Reaktionstemperatur relativ niedrig gehalten werden. Siehe EP-A-18 541. Damit ist jedoch der Nachteil verbunden, dass das nicht umgesetzte 4-Nitrotoluol aus der Sulfonierungsmasse mit Lösungsmitteln extrahiert werden muss. Will man einen Ausbeuteverlust vermeiden, muss das 4-Nitrotoluol aus dem Extrakt isoliert und in die Reaktion zurückgeführt werden. All dies kompliziert das Verfahren.

Den vorstehend geschilderten Nachteil versucht man mit dem Verfahren gemäss EP-A-83 555 zu vermeiden, indem man vor der Sulfonierung eine geringe Menge an Schwefelsäure zusetzt. Jedoch wie bei allen Verfahren, die mit gasförmigem $SO_3$ arbeiten, bereitet die Handhabung des letzteren gewisse Schwierigkeiten und erhöht den apparativen Aufwand. Wenn das $SO_3$ aus z.B. 65%igem Oleum gewonnen wird, bedeutet dies gegenüber dem direkten Sulfonieren mit Oleum einen zusätzlichen Prozessschritt.

Es sind noch etliche weitere Sulfonierungsverfahren mit $SO_3$ bekannt, in denen das $SO_3$ mit einem Inertgas verdünnt wird, um die exotherme Reaktion unter Kontrolle zu halten und das Problem der Bildung von Nebenprodukten, insbesondere von Sulfonen, zu vermeiden (diese Probleme treten bei Sulfonierung mit reinem $SO_3$ auf). Siehe z.B. DE-A-2 800 788, DE-A-2 413 444, JP-A-55-4357. Der Nachteil dieser Verfahrensweisen besteht jedoch generell darin, dass man erhebliche Mengen an Inertgas braucht und dass dann das Abgas von $SO_3$ oder von infolge von Feuchtigkeitszutritt gebildeter Schwefelsäure sowie auch von mitgerissenem Kohlenwasserstoff gereinigt werden muss, was einen beträchtlichen und unerwünschten Aufwand bedeutet.

Eine drastische Reduzierung der anfallenden Abfallschwefelsäuremenge im eingangs beschriebenen Standardverfahren (Sulfonierung von 4-Nitrotoluol mit 20-25%igem Oleum) könnte im Prinzip durch Erhöhung der Konzentration des $SO_3$ im Oleum erreicht werden, z.B. durch Verwendung des handelsüblichen 65%igen Oleums. Es ist jedoch bekannt, dass bei der Sulfonierung von aromatischen Kohlenwasserstoffen mit Oleum in zunehmendem Masse Nebenreaktionen auftreten, sobald die $SO_3$-Konzentration im Oleum erhöht wird. Diese Nebenreaktionen führen zur Schwarzfärbung der Sulfonierungsprodukte und damit auch zu Ausbeuteverminderungen. Diese Nebenreaktionen nehmen mit steigender Temperatur zu. Bei der Sulfonierung von 4-Nitrotoluol tritt dieses Problem besonders auf, da bei relativ hohen Temperaturen sulfoniert werden muss. Daher war es bis anhin nicht gelungen, ein wirtschaftliches und technisch brauchbares Sulfonierungsverfahren für 4-Nitrotoluol mit Oleum zu finden, das über 30% $SO_3$ enthält.

Es wurde nun überraschenderweise gefunden, dass man 4-Nitrotoluol auch mit über 50%igem Oleum problemlos und mit hohen Ausbeuten sulfonieren kann und hinreichend reine 4-Nitrotoluol-2-sulfonsäure erhält, wenn man die Sulfonierung kontinuierlich in einem Reaktionsgemisch ausführt, in dem der Umsetzungsgrad (Umsatz) von 4-Nitrotoluol $\geq$ 90% beträgt.

Das erfindungsgemässe Verfahren zur Herstellung von 4-Nitrotoluol-2-sulfonsäure durch Sulfonierung von 4-Nitrotoluol mit Oleum ist demnach dadurch gekennzeichnet, dass man die Sulfonierung kontinuierlich mit 50-85%igem Oleum durchführt und in der Reaktionsmasse, zu welcher 4-Nitrotoluol und Oleum zudosiert werden, den Umsetzungsgrad (Umsatz) von 4-Nitrotoluol während der gesamten Reaktionsdauer auf $\geq$ 90% hält.

Die Verweilzeit, die notwendig ist, um einen mindestens 90%igen 4-Nitrotoluol-Umsatz im Reaktionsgefäss (bzw. im ersten Reaktionsgefäss, wenn deren mehrere verwendet werden) zu erreichen bzw. aufrecht zu halten, kann in weiten Grenzen variieren und richtet sich nach der eingestellten Reaktionstemperatur und der zudosierten $SO_3$-Menge (als Oleum). Vorteilhaft wird bei Temperaturen von 80-140°C, vorzugsweise von 110-120°C, gearbeitet. Die entsprechenden Parameter (Dosiergeschwindigkeit von 4-Nitrotoluol und Oleum, Temperatur, Verweilzeit) werden in Vorversuchen bestimmt. Ob der 4-Nitrotoluol-Umsetzungsgrad von mindestens 90% eingehalten wird, wird zweckmässig während der gesamten Reaktionsdauer überprüft. Beispielsweise wird in bestimmten Zeitabständen eine Probe aus der Reaktionsmasse entnommen und darin die Menge an umgesetztem 4-Nitrotoluol analytisch bestimmt. Erforderlichenfalls kann einer oder mehrere der vorerwähnten Parameter nachreguliert werden.

Vorzugsweise wird pro Mol 4-Nitrotoluol 1,0-1,5 Mol $SO_3$ in Form von Oleum eingesetzt. Als besonders vorteilhaft hat sich ein leichter stöchiometrischer Überschuss an $SO_3$, z.B. 1,05-1,1 Mol pro Mol 4-Nitrotoluol, erwiesen.

Man verwendet als Sulfonierungsmittel beispielsweise 50-80%iges Oleum. In der Praxis setzt man insbesondere 60-70%iges, vorzugsweise 65%iges Oleum (= 65% $SO_3$-Gehalt) ein, das im Handel in dieser Konzentration erhältlich ist. Das Verfahren ist aber auch sehr vorteilhaft mit höher konzentriertem Oleum durchführbar, etwa mit 70-85%igem, beispielsweise 81%igem oder 85%igem Oleum.

Das erfindungsgemässe Verfahren kann in allen in der chemischen Verfahrenstechnik bekannten, für kontinuierlich geführte Reaktionen geeigneten Vorrichtungen und Apparaturen durchgeführt werden. Als vorteilhaft zur Durchführung des Verfahrens hat sich ein Durchflussrührkessel, insbesondere eine Rührkesselkaskade, erwiesen. Das erfindungsgemässe Verfahren kann aber z.B. auch in einer Kreislaufapparatur, etwa einem Schlaufenreaktor, durchgeführt werden. Gegebenenfalls können Nachreaktoren vorgesehen werden, beispielsweise Verweilzeitgefässe, ein Strömungsrohr oder eine Rührkesselkaskade.

Besonders bevorzugt wird das Verfahren in einer Rührkesselkaskade mit einem Hauptreaktionskessel und Nachreaktionskesseln ausgeführt.

Um beim erstmaligen Anfahren der Reaktion die Bildung von Nebenprodukten (Schwarzfärbung) aus 4-Nitrotoluol und dem konzentrierten Oleum zu vermeiden, kann im (ersten) Reaktionsgefäss Sulfonierungsmasse mit mindestens 90%igen 4-Nitrotoluol-Umsatz (z.B. ausreagierte Sulfonierungsmasse) vorgelegt und anschliessend 4-Nitrotoluol und Oleum zudosiert werden.

Es kann jedoch auch konzentrierte Schwefelsäure vorgelegt und anschliessend 4-Nitrotoluol und Oleum gleichzeitig zudosiert werden, wobei durch die anfängliche Oleum-Verdünnung eine Schwarzfärbung vermieden wird. In der einen Überschuss an Schwefelsäure enthaltenden Reaktionsmischung stellt sich sehr schnell ein 4-Nitrotoluol-Umsetzungsgrad von > 90% ein, womit die im erfindungsgemässen Verfahren notwendige Bedingung erfüllt ist.

Je nach beabsichtiger Weiterverwendung kann die Aufarbeitung des Reaktionsgemisches nach verschiedenen Methoden erfolgen. Das erfindungsgemässe Verfahren liefert 4-Nitrotoluol-2-sulfonsäure in guter Ausbeute (98-99,5% der Theorie) und hoher Reinheit (wenig Nebenprodukte, z.B. 2,2'-Dimethyl-5,5'-dinitrodiphenylsulfon). Die Reaktionsmasse kann daher z.B. direkt weiterverwendet werden, z.B. zur Herstellung von 4,4'-Dinitrostilben-2,2'-disulfonsäure. Besonders vorteilhaft kann sie mit Wasser verdünnt werden, z.B. auf einen Gehalt von 30-35% an 4-Nitrotoluol-2-sulfonsäure, und kann dann im Herstellungsverfahren für 4,4'-Dinitrostilben-2,2'-disulfonsäure eingesetzt werden. Falls die in der Reaktionsmasse vorhandene Schwefelsäure bei der Weiterverarbeitung stört, kann die 4-Nitrotoluol-2-sulfonsäure auch isoliert werden.

Dies geschieht zweckmässig durch Verdünnen der Sulfonierungsmasse, z.B. auf eine Schwefelsäurekonzentration von 60-75%, insbesondere etwa 70%, und auf eine 4-Nitrotoluol-2-sulfonsäure-Konzentration von 30-40%, insbesondere etwa 35%. Die 4-Nitrotoluol-2-sulfonsäure kristallisiert dabei aus und kann in üblicher Weise abgetrennt werden. Die Verdünnung zur entsprechenden Schwefelsäure- und 4-Nitrotoluol-2-sulfonsäure-Konzentration kann durch Wasser erfolgen. Bevorzugt jedoch wird, um die Menge an Abfallschwefelsäure gering zu halten, die Verdünnung mit Wasser und Mutterlauge aus vorangegangenen Kristallisationsschritten durchgeführt (Recylierung der schwefelsauren Mutterlaugen). Zu diesem Zweck kann z.B. die Reaktionsmasse zuerst mit Wasser auf die oben angegebene gewünschte Schwefelsäurekonzentration, und dann mit Mutterlauge aus einem vorangegangenen Kristallisationsschritt auf die gewünschte 4-Nitrotoluol-2-sulfonsäure-Konzentration verdünnt werden. Es kann aber auch die berechnete Menge an Wasser und Mutterlauge vorgelegt und die Reaktionsmasse dieser Mischung zugegeben werden, worauf sich die gewünschte Konzentration an Schwefelsäure und 4-Nitrotoluol-2-sulfonsäure einstellt und letztere auskristallisiert.

Das erhaltene Produkt ist sehr rein und kann ohne weitere Reinigungsoperation weiterverwendet werden. Da nur sehr geringe Mengen an Sulfon enthalten sind, ist eine Abtrennung des letzteren nicht erforderlich.

Wie bereits eingangs erwähnt, wird der Grossteil der erzeugten 4-Nitrotoluol-2-sulfonsäure zur Herstellung von 4,4'-Dinitrostilben-2,2'-disulfonsäure verwendet. Zu diesem Zweck wird beispielsweise eine ca. 30-50%ige wässrige Lösung von 4-Nitrotoluol-2-sulfonsäure mit Oxidationsmitteln (z.B. Luftsauerstoff, Hypochlorit usw.) umgesetzt. Die erhaltene 4,4'-Dinitrostilben-2,2'-disulfonsäure kann dann zur 4,4'-Diaminostilben-2,2'-disulfonsäure reduziert werden (siehe z.B. Ber. 30, 3100; EP-A-83 555, S. 11/12, 15/16) und ist ein wichtiges Zwischenprodukt für die Herstellung von optischen Aufhellern [siehe z.B. Angew. Chem. 87, 693 (1975)].

In den nachfolgenden Beispielen werden besonders bevorzugte Ausführungsformen des erfindungsgemässen Verfahrens beschrieben. Es wird jedoch betont, dass die Erfindung nicht auf diese Beispiele beschränkt ist.

Sofern nichts anderes angegeben ist, beziehen sich in der gesamten Beschreibung und in den Patentansprüchen Teile- und Prozentangaben auf das Gewicht.

### Beispiel 1

In den ersten Rührkessel einer 4stufigen Rührkesselkkaskade mit den Volumen 800, 400, 400, 400 ml werden bei 115°C zuerst 200 g Schwefelsäure 100% vorgelegt und anschliessend gleichzeitig 600 g 4-Nitrotoluol (4,376 Mol) und 566 g Oleum 65% (4,595 Mol SO$_3$) pro Stunde kontinuierlich zudosiert. Der 4-Nitrotoluol-Umsatz im ersten Rührkessel beträgt im stationären Zustand 93%. In den folgenden Rührkesseln erfolgt die vollständige Umsetzung des 4-Nitrotoluols zur 4-Nitrotoluol-2-sulfonsäure bei 115°C. Die Verweilzeit in der Kaskade beträgt 2$^1$/$_2$ Stunden.

Zur Isolierung der 4-Nitrotoluol-2-sulfonsäure wird die Sulfonierungsmasse mit Wasser auf eine Schwefelsäurekonzentration von 70% eingestellt und mit Mutterlauge einer vorangegangenen Kristallisation auf eine 4-Nitrotoluol-2-sulfonsäure-Konzentration von 35% verdünnt. Nach Zentrifugieren bei Raumtemperatur erhält man aus 600 g 4-Nitrotoluol-2-sulfonsäure 100%, entsprechend 98,5% d.Th.

Anstelle von 200 g Schwefelsäure 100% kann im ersten Rührkessel auch eine entsprechende Menge an ausreagierter Sulfonierungsmasse aus einer vorangegangenen Fabrikationsphase vorgelegt werden.

### Beispiel 2

Es wird die im Beispiel 1 beschriebene Rührkesselkaskade verwendet. In den ersten Rührkessel werden 200 g Schwefelsäure 100% vorgelegt und anschliessend bei 115°C gleichzeitig 600 g 4-Nitrotoluol (4,376 Mol) und 454 g Oleum 85% (4,814 Mol SO$_3$) pro Stunde kontinuierlich zudosiert. Das Oleum 85% wird durch Mischen von Oleum 65% und SO$_3$ 100% hergestellt und mit einer Temperatur von 50°C aus einem Druckgefäss zudosiert. Der 4-Nitrotoluol-Umsatz im ersten Rührkessel beträgt im stationären Zustand 92%. Die Verweilzeit in der Kaskade beträgt 170 Minuten.

Die Sulfiermasse wird durch Zugabe von Wasser auf eine 4-Nitrotoluol-2-sulfonsäure-Konzentration von 35% verdünnt. Im stationären Betrieb erhält man aus 600 g 4-Nitrotoluol durch Zugabe von 1635 g Wasser ca. 2690 g 4-Nitrotoluol-2-sulfonsäure-Lösung mit einem Gehalt von 35 Gew.-%. Das entspricht 941 g 4-Nitrotoluol-2-sulfonsäure 100% oder 99,0% d.Th. (bezogen auf 4-Nitrotoluol).

Die erhaltene 4-Nitrotoluol-2-sulfonsäure-Lösung mit einem Schwefelsäure-Gehalt von ca. 4% wird direkt zur Weiterverarbeitung (Herstellung von 4,4'-Dinitrostilben-2,2'-disulfonsäure) eingesetzt.

### Patentansprüche

1. Verfahren zur Herstellung von 4-Nitrotoluol-2-sulfonsäure durch Sulfonierung von 4-Nitrotoluol mit Oleum, dadurch gekennzeichnet, dass die Sulfonierung mit 50-85%igem Oleum kontinuierlich durchgeführt und in der Reaktionsmasse, zu welcher 4-Nitrotoluol und Oleum zudosiert werden, der Umsetzungsgrad von 4-Nitrotoluol während der gesamten Reaktionsdauer auf ≥ 90% gehalten wird.

2. Verfahren nach Anspruch 1, dadurch gekennzeichnet, dass man zum Anfahren der kontinuierlichen Reaktion ausreagierte Sulfonierungsmasse vorlegt und anschliessend 4-Nitrotoluol und Oleum zudosiert.

3. Verfahren nach Anspruch 1, dadurch gekennzeichnet, dass man zum Anfahren der kontinuierlichen Reaktion vorerst konzentrierte Schwefelsäure vorlegt und anschliessend 4-Nitrotoluol und Oleum gleichzeitig zudosiert.

4. Verfahren nach einem der Ansprüche 1 bis 3, dadurch gekennzeichnet, dass die Sulfonierung bei Temperaturen von 80-140°C, vorzugsweise von 110-120°C, erfolgt.

5. Verfahren nach einem der Ansprüche 1 bis 4, dadurch gekennzeichnet, dass pro Mol 4-Nitrotoluol 1,0-1,5 Mol, vorzugsweise 1,05-1,1 Mol, SO$_3$ in Form von Oleum eingesetzt werden.

6. Verfahren nach einem der Ansprüche 1 bis 5, dadurch gekennzeichnet, dass man mit 50-80%igem, vorzugsweise 60-70%igem Oleum sulfoniert.

7. Verfahren nach einem der Ansprüche 1 bis 5, dadurch gekennzeichnet, dass man mit 70-85%igem Oleum sulfoniert.

8. Verfahren nach einem der Ansprüche 1 bis 7, dadurch gekennzeichnet, dass man die Sulfonierung in einem Durchlaufrührkessel durchführt.

9. Verfahren nach Anspruch 8, dadurch gekennzeichnet, dass man im Durchlaufrührkessel den Umsetzungsgrad von 4-Nitrotoluol auf ≥ 90% einstellt und 4-Nitrotoluol und Oleum kontinuierlich zudosiert.

10. Verfahren nach Anspruch 8, dadurch gekennzeichnet, dass die Reaktion in einer angeschlossenen Rührkesselkaskade zu Ende geführt wird.

11. Verfahren nach Anspruch 8, dadurch gekennzeichnet, dass die Reaktion in Verweilzeitgefässen zu Ende geführt wird.

12. Verfahren nach Anspruch 8, dadurch gekennzeichnet, dass die Reaktion in einem Strömungsrohr zu Ende geführt wird.

13. Verfahren nach einem der Ansprüche 1 bis 7, dadurch gekennzeichnet, dass man die Sulfonierung in einem Schlaufenreaktor durchführt.

14. Verfahren nach einem der Ansprüche 1-13, dadurch gekennzeichnet, dass man zur Isolierung der 4-Nitrotoluol-2-sulfonsäure aus der Reaktionsmasse letztere mit Wasser und Mutterlauge aus einem vorausgegangenen 4-Nitrotoluol-2-sulfonsäure-Kristallisationsschritt verdünnt und auf eine Schwefelsäurekonzentration von 60-75% und eine 4-Nitrotoluol-2-sulfonsäure-Konzentration von 30-40% einstellt und die auskristallisierte 4-Nitrotoluol-2-sulfonsäure abtrennt.

15. Verfahren nach einem der Ansprüche 1-13, dadurch gekennzeichnet, dass man die erhaltene Sulfonierungsmasse, gegebenenfalls nach Verdünnung mit Wasser z.B. auf einen ca. 30-50%igen Gehalt an 4-Nitrotoluol-2-sulfonsäure, mit Oxidationsmitteln direkt weiter zu 4,4'-Dinistrostilben-2,2'-disulfonsäure umsetzt.


## Claims

1. A process for the preparation of 4-nitrotoluene-2-sulfonic acid by sulfonating 4-nitrotoluene with oleum, which comprises carrying out the sulfonation continuously with 50-85% oleum and keeping the conversion in the reaction mixture, to which 4-nitrotoluene and oleum are added, at ≥ 90% during the entire reaction course.

2. A process according to claim 1, which comprises charging the reactor with a fully reacted sulfonation batch to initiate the continuous reaction and then adding 4-nitrotoluene and oleum.

3. A process according to claim 1, which comprises charging the reactor first with concentrated sulfuric acid to initiate the continuous reaction and then simultaneously adding 4-nitrotoluene and oleum.

4. A process according to any one of claims 1 to 3, wherein the sulfonation is carried out in the temperature range from 80° to 140°C, preferably from 110° to 120°C.

5. A process according to any one of claims 1 to 4, wherein 1.0 to 1.5 moles, preferably 1.05 to 1.1 moles, of SO3 in the form of oleum are used per mole of 4-nitrotoluene.

6. A process according to any one of claims 1 to 5, wherein the sulfonation is carried out with 50 to 80%, preferably 60-70%, oleum.

7. A process according to any one of claims 1 to 5, wherein the sulfonation is carried ou with 70-85% oleum.

8. A process according to any one of claims 1 to 7, wherein the sulfonation is carried out in a stirred flow reactor.

9. A process according to claim 8, wherein the conversion of 4-nitrotoluene in the stirred flow reactor is adjusted to ≥ 90% and 4-nitrotoluene and oleum are continuously added.

10. A process according to claim 8, wherein the reaction is brought to completion in a connected cascade of agitator vessels.

11. A process according to claim 8, wherein the reaction is brought to completion in residence time vessels.

12. A process according to claim 8, wherein the reaction is brought to completion in a tubular reactor.

13. A process according to any one of claims 1 to 7, wherein the sulfonation is carried out in a loop reactor.

14. A process according to any one of claims 1 to 13, wherein the 4-nitrotoluene-2-sulfonic acid is isolated from the reaction mixture by diluting the reaction mixture with water and mother liquor from a previous 4-nitrotoluene-2-sulfonic acid crystallisation step and adjusting it to a sulfuric acid concentration of 60-75% and to a 4-nitrotoluene-2-sulfonic acid concentration of 30-40% and separating the crystallised 4-nitrotoluene-2-sulfonic acid.

15. A process according to any one of claims 1 to 13, which comprises reacting the sulfonation batch further, optionally after dilution with water, e.g. to an approximately 30-50% content of 4-nitrotoluene-2-sulfonic acid, with oxidising agents direct to 4,4'-dinistrostilbene-2,2'-disulfonic acid.


## Revendications

1. Procédé pour la préparation de l'acide 4-nitrotoluène-2-sulfonique par sulfonation du 4-nitrotoluène avec de l'oléum, caractérisé par le fait que l'on effectue la sulfonation en continu avec de l'oléum à 50-85% et, pendant toute la durée de la réaction, on maintient le taux de conversion du 4-nitrotoluène à ≥ 90% dans la masse réactionnelle, dans laquelle le 4-nitrotoluène et l'oléum sont introduits de façon réglée.

2. Procédé selon la revendication 1, caractérisé par le fait que pour le déclenchement de la réaction continu, on dispose au préalable une masse de sulfonation ayant cessé de réagir, et on y ajoute ensuite de façon réglée du 4-nitrotoluène et de l'oléum.

3. Procédé selon la revendication 1, caractérisé par le fait que pour le déclenchement de la réaction continue, on dispose en premier lieu de l'acide sulfurique concentré, et on ajoute ensuite de façon réglée, simultanément, du 4-nitrotoluène et de l'oléum.

4. Procédé selon l'une des revendications 1 à 3, caractérisé par le fait que l'on effectue la sulfonation à des températures allant de 80 à 140°C, de préférence de 110 à 120°C.

5. Procédé selon l'une des revendications 1 à 4, caractérisé par le fait que l'on utilise, par mole de 4-nitrotoluène, 1,0-1,5 mole, de préférence 1,05 à 1,1 mole de SO3 sous forme d'oléum.

6. Procédé selon l'une des revendications 1 à 5, caractérisé par le fait que l'on effectue la sulfonation avec de l'oléum à 50-80%, de préférence à 60-70%.

7. Procédé selon l'une des revendications 1 à 5, caractérisé par le fait que l'on effectue la sulfonation avec de l'oléum à 70-85%.

8. Procédé selon l'une des revendications 1 à 7, caractérisé par le fait que l'on effectue la sulfonation dans une cuve à écoulement continu et à agitation.

9. Procédé selon la revendication 8, caractérisé par le fait que, dans la cuve à écoulement continu et à agitation, on règle le taux de conversion du 4-nitrotoluène à ≥ 90% et on ajoute en continu, de façon réglée, du 4-nitrotoluène et de l'oléum.

10. Procédé selon la revendication 8, caractérisé par le fait que l'on achève la réaction dans une cascade de cuves à agitation se faisant suite.

11. Procédé selon la revendication 8, caractérisé par le fait que l'on achève la réaction dans des récipients de retenue.

12. Procédé selon la revendication 8, caractérisé par le fait que l'on achève la réaction dans un tube à écoulement.

13. Procédé selon l'une des revendications 1 à 7, caractérisé par le fait que l'on effectue la sulfonation dans un réacteur à boucle.

14. Procédé selon l'une des revendications 1 à 13, caractérisé par le fait que pour l'isolement de l'acide 4-nitrotoluène-2-sulfonique hors de la masse réactionnelle, on dilue celle-ci avec de l'eau et de la liqueur-mère provenant d'une étape précédente de cristallisation de l'acide 4-nitrotoluène-2-sulfonique et on ajouste le mélange à une concentration d'acide sulfurique de 60-75% et une concentration d'acide 4-nitrotoluène-2-sulfonique de 30-40%, puis on isole l'acide 4-nitrotoluène-2-sulfonique séparé en cristaux.

15. Procédé selon l'une des revendications 1 à 13, caractérisé par le fait que l'on fait directement réagir à nouveau, avec des oxydants, la masse obtenue après la sulfonation, éventuellement après dilution avec de l'eau, par exemple à une teneur d'environ 30-50% en acide 4-nitrotoluène-2-sulfonique, pour aboutir à l'acide 4,4'-dinitrostilbène-2,2'-disulfonique.